# EUROPEAN PATENT APPLICATION

(11) **EP 2 272 487 A1**
(43) Date of publication of application: **12.01.2011**
(21) Application number: 09008926.9
(22) Date of filing: 08.07.2009
(51) Int. Cl.: A61K 6/083, A61L 24/12, C08G 75/02, C08G 83/00

(54) **Process for the preparation of a composition comprising hyperbranched compounds**

(71) Applicant: Dentsply DeTrey GmbH, 78467 Konstanz (DE)
(72) Inventor: Klee, Joachim E., 78315 Radolfzell (DE); Ritter, Helmut, 42111 Wuppertal (DE); Pohle, Sven, 78467 Konstanz (DE); Bezdushna, Ella, 40589 Düsseldorf (DE); Elsner, Oliver, 79790 Küssaberg (DE)
(74) Representative: Hartz, Nikolai

(57) **Abstract**

A process for the preparation of a composition comprising hyperbranched polymeric compounds, which comprises a step of reacting a mixture comprising one or more compounds of the following formula (I):

AR(B)ₙ

wherein
A and B are functional groups,
R is an (n+1)-valent organic group containing one or more thioether groups and
n is an integer of at least 2
**characterized in that**
A is an amino group or a hydroxyl group, and
B is a carboxylic acid group or an ester or anhydride thereof,
under reaction conditions wherein A reacts with B and forms a linking amide group, while A does not react with A and B does not react with B.

## Description

### Field of the Invention

The present invention relates to a process for the preparation of a composition comprising hyperbranched polymeric compounds which may be incorporated into a dental composition. The present invention also relates to the use of a composition comprising the hyperbranched polymeric compounds for the preparation of a dental cement.

### Background of the Invention

Dental cements are usually powder liquid systems consisting of linear poly(alkenoic acid)s and reactive ion releasing active glasses. The most common poly(alkenoic acid)s are polymers such as polyacrylic acid or copolymers of acrylic and itaconic acid, acrylic acid and maleic acid and to some degree a copolymer of acrylic acid with methacrylic acid.

In the presence of water, the poly(alkenoic acid) attacks the glass powder whereby metal ions such as calcium, aluminum and strontium are released under formation of intra- and intermolecular salt bridges which crosslink the composition.

Generic cements have a number of important advantages for applications in dentistry such as the virtual absence of an exothermic reaction, no shrinkage during setting, no free monomer in the set composition, high dimensional stability, fluoride release and good adhesion to tooth structure.

Beside these advantageous properties, the main limitation of the glass ionomer cements is their relative lack of strength and low resistance to abrasion and wear. Conventional glass ionomer cements have low flexural strength but high modulus of elasticity, and are therefore very brittle and prone to bulk fracture.

In order to improve the mechanic properties especially flexural strength and fracture toughness numerous investigations were carried out in the last decades, which are directed to the use of amino acids (Z. Ouyang, S. K. Sneckberger, E. C. Kao, B. M. Culbertson, P. W. Jagodzinski, Appl. Spectros 53 (1999) 297-301; B. M. Culbertson, D. Xie, A. Thakur, J. Macromol. Sci. Pure Appl. Chem. A 36 (1999) 681-96), the application of water soluble copolymers using poly(N-vinylpyrrolidone) (D. Xie, B. M. Culbertson, G. J. Wang, J. Macromol. Sci. Pure Appl. Chem. A 35 (1998) 54761), the use of polyacids with narrow molecular weight distribution (DE 100 58 829) and star-like branched polyacids (DE 100 58 830). Further polyacids having a limited molecular mass ranging from 20,000 to 50,000 D (EP 0 797 975) and 1,000 to 50,000 D (WO 02/41845) were proposed. A further approach was the application of spherical ionomer particles (WO 00/05182).

EP 1 600 142 discloses dental cement compositions containing composite particles with grafted polyacidic polymer chains. WO 02/41846 discloses the use of branched polyacids in dental compositions. EP 1337 221 discloses the use of branched polyacids in dental compounds. However, polyacids suggested according to these reference have an average branch length which is similar to the overall degree of polymerization.

Dendrimers, arborols, starburst polymers, and hyperbranched polymers are designations for polymeric structures which are distinguished by a branched structure and a high functionality. Among such polymers, hyperbranched polymers possess both molecular and structural nonuniformity (Nachrichten aus Chemie, Technik und Laboratorium, 2002, 50, 1218; Dendrimers and Dendrons, Concepts, Syntheses, Applications by G. R. Newkome, C. N. Moorefield, F. Vögtle, Wiley-VCH, 2001, Rev. Macromol. Chem. 1997, C37(3), 555). Therefore, molecular weight and functionality of the hyperbranched polymers are known to be problematic for many technical applications. Moreover, due to the complicated multistep synthesis inherently required for the preparation of dendrimers, an application in practice is inefficient and costly.

### Summary of the Invention

It is an object of the present invention to provide a process for the preparation of a composition for a novel dental cement systems setting by a cement reaction whereby the cured cement has improved flexural strength and fracture toughness.

This problem is solved according to the invention by a process for the preparation of a composition comprising hyperbranched polymeric compounds, which comprises a step of reacting a mixture comprising one or more compounds of the following formula (I):

AR(B)ₙ

wherein
A and B are functional groups,
R is an (n+1)-valent organic group containing one or more thioether groups and
n is an integer of at least 2
**characterized in that**
A is an amino group or a hydroxyl group, and
B is a carboxylic acid group or an ester or anhydride thereof,
under reaction conditions wherein A reacts with B and forms a linking amide or ester group, while A does not react with A and B does not react with B.

The present invention is based on the recognition that the mechanical properties of dental cements may be significantly improved by using cement compositions containing hyperbranched polymeric compounds as polyacidic polymer chains as a component of the cement reaction. Accordingly, the present invention provides hyperbranched polymeric compounds for a novel dental cement which sets by a cement reaction.

According to the invention hyperbranched polymeric compounds are prepared based on AR(B)ₙ molecules having two different functional groups, A and B, which are able to react with one another to form a linking amide or ester groups. The functional group A is present in the molecule only once, the group B at least twice, i.e. n is an integer greater than or equal to 2.

The reaction of the AR(B)ₙ molecules with one another produces uncrosslinked, hyperbranched polymeric compounds having regularly arranged branching sites. The composition of the present invention can be used for the preparation of novel dental cement systems setting by a cement reaction whereby the cured cement has improved flexural strength and fracture toughness based on ionic crosslinking of the reactive glass filled by the hyperbranched polymeric compounds. The mechanical properties may be further improved by subsequent covalent crosslinking of the cement composition by appropriate addition or condensation reactions based on functional groups present in the hyperbranched polymeric compounds.

### Description of the Preferred Embodiments

The present invention provides a process for the preparation of a composition comprising hyperbranched polymeric compounds. The hyperbranched polymeric compounds according to the present invention preferably have an average branch length which is small as compared to the overall degree of polymerization of the alkenoic acid monomers used for the preparation of the hyperbranched polymeric compounds.

Preferably, the hyperbranched polymeric compounds according to the present invention have a molecular weight in the range of from 20,000 to 2,000,000, more preferably in the range of from 100,000 to 500,000.

The process for the preparation of a hyperbranched polymeric compounds according to the present invention comprises a step of reacting a mixture comprising one or more compounds of formula (I) as defined above.

According to formula (I), A and B are functional groups.

Specifically, A may be an amino group or a hydroxyl group. Preferably, A is an amino group.

B is a carboxylic acid group, or an ester or anhydride thereof. An ester group may be a group wherein B is -COOR¹⁰, wherein R¹⁰ is a straight-chain or branched C₁ to C₈ alkyl or a straight-chain or branched C₃ to C₈ cycloalkyl group. An anhydride group may be a group wherein B is -COOCOR¹¹, wherein R¹¹ is a straight-chain or branched C₁ to C₈ alkyl or a straight-chain or branched C₃ to C₈ cycloalkyl group.

According to formula (I), R is an (n+1)-valent organic group containing one or more thioether groups. R may be a hydrocarbon residue. Preferably, R contains heteroatoms such as oxygen, nitrogen or sulfur. R may contain further functional groups which may undergo addition or condensation reactions. According to formula (I), n is an integer of at least 2. Preferably n is 2 to 10, more preferably 2 to 4.

In a preferred embodiment, the one or more compounds of formula (I) comprise a compound obtainable by telomerizing a mixture containing one or more polymerizable unsaturated carboxylic acid monomers or esters or anhydrides thereof with a compound containing a group A and one or more SH-groups.

The compound containing a group A and one or more SH-groups may be a compound of the following formula (II)

Y-L¹-X(L²SH)ₓ

wherein
- Y: is an amino group or a hydroxyl group, preferably an amino group;
- L₁: is a linking group,
- L₂: is a linking group,
- X: is a single bond, O S, NR^{a}, -N< -CR^{a}R^{b}, or -CR^{a}< or >C<,
- x: is an integer of from 1 to 3 so that the valencies of X is (x+1), and
- R^{a} and R^{b}: are independent from each other hydrogen, a carboxylic acid group when linked to a carbon atom, or an alkyl group.

In case x is 2 or 3, a compound of formula (I) will contain additional branching sites.

A linking group may be a substituted or unsubstituted C₁ to C₁₈ alkyl group, a substituted or unsubstituted C₃ to C₈ cycloalkyl group, a substituted or unsubstituted C₄ to C₁₈ aryl or heteroaryl group, a substituted or unsubstituted C₅ to C₁₈ alkylaryl or alkylheteroaryl group, or a substituted or unsubstituted C₇ to C₃₀ aralkyl group.

Preferably, the one or more polymerizable unsaturated carboxylic acid monomers are one or more free radically polymerizable monomers containing optionally protected acidic groups including carboxylic acid groups. Suitable monomers for the polymerization process of the invention contain carboxylic acid groups optionally in protected form, and a polymerisable double bond. The acidic groups are selected from carboxylic acid groups, sulfonic acid groups, sulfuric acid groups, phosphonic acid groups, and phosphoric acid groups.

Preferably, the radically polymerizable monomer is a monomer of the following formula (II) 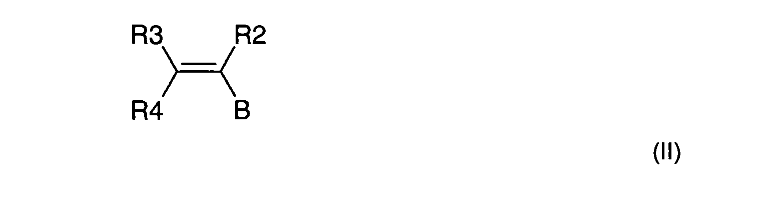 wherein B is a moiety containing a carboxylic acid group which may optionally be protected, and optionally a spacer group such as an alkylene group; R2 is a hydrogen atom, a carboxyl group, an C₁₋₆ alkyl group which may be substituted by a carboxyl group or a C₃₋₆ cycloalkyl group which may be substituted by a carboxyl group, and R3 and R4, which may be the same or different from each other, represent a hydrogen atom, a carboxyl group, an C₁₋₆ alkyl group which may be substituted by a carboxyl group or a C₃₋₆ cycloalkyl group which may be substituted by a carboxyl group. The carboxyl groups in R2, R3 or R4 may optionally be protected or form intramolecular anhydride moieties with adjacent carboxylic acid groups.

B is preferably a carboxyl group. R2 is preferably a hydrogen atom or a methyl group. R3 and R4 are preferably independent from each other hydrogen atoms, carboxyl groups, or an C₁₋₃ alkyl group which may be substituted by a carboxyl group.

Specific examples for a monomer of formula (II) are acidic monomers such as acrylic acid, methacrylic acid, maleic acid, fumaric acid, maleic acid anhydride, itaconic acid or itaconic acid anhydride. Preferably, the unsaturated carboxylic acid derivative may be an optionally protected acrylic acid or methacrylic acid such as tert.-butyl (meth)acrylic acid or n-butyl (meth)acrylic acid.

The protecting group for the carboxylic acidic group may be any suitable protecting group conventionally used for a respective carboxylic acidic group. The protecting group is advantageously selected so as to be removable after the polymerization reaction. Preferably, the liberated protecting group does not have any adverse effects on the human body. A preferred protecting group especially for a carboxyl group is a tert.-butyl group or a n-butyl group.

The radically polymerizable optionally protected acid functional monomers can be polymerized optionally in the presence of other polymerisable monomers.

According to a preferred embodiment, thioether groups are obtainable by a reaction according to the following reaction scheme: 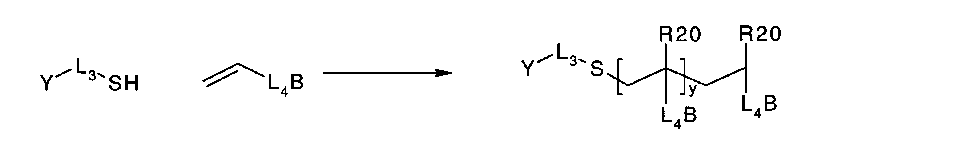 wherein Y is an amino group or a hydroxyl group, L₃ and L₄ are independent from each other linking groups, R²⁰ is a hydrogen atom or a straight-chain or branched C₁ to C₈ alkyl or a straight-chain or branched C3 to C8 cycloalkyl group, y is an integer of from 10 to 10,000 and B is as defined above. If necessary, the amino group may be protected by a suitable protecting group.

A linking group may be a substituted or unsubstituted C₁ to C₁₈ alkyl group, a substituted or unsubstituted C₃ to C₈ cycloalkyl group, a substituted or unsubstituted C₄ to C₁₈ aryl or heteroaryl group, a substituted or unsubstituted C₅ to C₁₈ alkylaryl or alkylheteroaryl group, or a substituted or unsubstituted C₇ to C₃₀ aralkyl group.

The telomerization reaction of one or more compounds of formula (II) may be carried out as an aqueous chain transfer polymerization using a functional chain transfer agent and initiator. A preferred chain transfer reagent is cysteamine. A preferred initiator is ammonium persulfate. Accordingly, a monomer solution of one or more compounds of formula (II) may be prepared in distilled water. After deaeration, the chain transfer reagent and the initiator are added. The telomerization may be carried out at a temperature in the range of from more than 0°C to less than 100°, preferably in the range of from 10 to 50 °C. The reaction time is not specifically limited and may be selected from 1 hour to 48 hours, preferably 10 hours to 36 hours. The telomerized product may be separated and purified by dialysis followed by lyophilizaiton in order to obtain a polyacid containing terminal amino groups.

According to the present invention, the compounds of formula (I) are reacted under reaction conditions wherein A reacts with B and forms a linking amide group, while A does not react with A and B does not react with B.

Preferably, the reaction conditions include subjecting the reaction mixture to microwave irradiation. Under such conditions compounds of formula (I) form hyperbranched structures. Accordingly, the polyacid containing terminal amino groups may be hyperbranched by placing the polyacid containing terminal amino groups in a pressure-resistant reaction vessel provided with a magnetic stirring bar. The reaction vessel is advantageously placed in a microwave apparatus. Microwave irradiation is applied at a power of from 1 to 1000 W, preferably 10 to 100 W at a temperature in the range of from 10 to 200 °C for 30 seconds to 3 hours, preferably 5 to 60 minutes. The hyperbranched product may be separated and purified by lyophilizaiton in order to obtain a hyperbranched polyacid.

The polyacid containing terminal amino groups may be hyperbranched in the presence of a further polyacid, such as a polyacrylic acid. Accordingly, the reaction components are thoroughly mixed as fine powder in a porcelain cup. The mixture is preferably placed in a pressure-resistant reaction vessel provided with a magnetic stirring bar. The reaction vessel is then placed in a microwave apparatus. Microwave irradiation is applied at a power of from 1 to 1000 W, preferably 10 to 100 W at a temperature in the range of from 10 to 200 °C for 30 seconds to 3 hours, preferably 5 to 60 minutes. The hyperbranched product may be separated and purified by lyophilizaiton in order to obtain a hyperbranched polyacid.

By using the process, a hyperbranched polymeric compound is formed. The hyperbranched polymeric compounds contain carboxylic acidic groups and/or protected acidic groups, and optionally further functional groups. In case the hyperbranched polymeric compounds contain protected groups, it is preferred to deprotect protected acidic groups, for forming hyperbranched polymeric compounds with hyperbranched polyacidic polymer chains.

The process for the preparation of the hyperbranched polymeric compounds according to the invention provides compounds with a large number or functional groups which may be available for transformation into another functional group or moiety after the desired hyperbranched structure is formed.

A transformation may be a condensation or addition reaction. The condensation reaction or addition reaction may provide polymerizable double-bonds so that the hyperbranched compounds obtainable according to the present invention may not only be used as components in a cement reaction with a glass ionomer component, but also as polymerisable component in an additional polymerization reaction. Accordingly, the present invention further provides a process for further modification of the hyperbranched polymers of the invention for providing modified and/or covalently crosslinked hyperbranched polymeric compounds of the invention. Modified and/or covalently crosslinked polymeric compounds of the invention may be prepared from hyperbranched polymeric compounds of the invention by reaction with a bifunctional or multifunctional compound, e.g. with at least one polyhydric alcohol or with at least one alkanolamine or with a vinyl ether or aminoalkylthiol, or hydroxy(meth) acrylic acid.

Examples that may be mentioned of polyhydric alcohols used with preference include the following: alcohols having at least 2 hydroxyl groups, such as ethylene glycol, 1,2-propanediol, 1,4-butanediol, 1,3-propanediol, 1,2-butanediol, glycerol, butane-1,2,4-triol, n-pentane-1,2,5-triol, n-pentane-1,3,5-triol, n-hexane-1,2,6-triol, n-hexane-1,2,5-triol, n-hexane-1,3,6-triol, trimethylolbutane, trimethylolpropane or ditrimethylolpropane, trimethylolethane, pentaerythritol or dipentaerythritol; sugar alcohols such as mesoerythritol, threitol, sorbitol, mannitol or mixtures of the aforementioned alcohols.

Alkanolamines include monoalkanolamines, N,N-dialkylalkanolamines, N-alkylalkanolamines, dialkanolamines, N-alkylalkanolamines, and trialkanolamines, each having 2 to 18 carbon atoms in the hydroxyalkyl radical and, where appropriate, 1 to 6 carbon atoms in the alkyl radical, preferably 2 to 6 carbon atoms in the alkanol radical and, where appropriate, 1 or 2 carbon atoms in the alkyl radical.

The polymerization process according to the invention may further comprise a step of isolating hyperbranched polymeric compounds.

In a further embodiment, the mixture subjected to hyperbranching may further comprise a polyacrylic acid molecule or an anydride thereof having an average molecular weight of from 0.5 to 500 kDa, preferably 1 to 200 kDa, more preferably 10 to 150 kDa.

The present invention provides, furthermore, for the use of the hyperbranched polymeric compounds of the invention and of the polyaddition or polycondensation products prepared from the hyperbranched polymers of the invention as a component of dental compositions, notably dental cements.

A dental cement composition provided according to the present invention comprises a particulate reactive inorganic filler capable of leaching metal ions in the presence of an acid and water. The filler is preferably a reactive glass capable of leaching metal ions and advantageously also fluoride ions. The reactive glass may be any glass ionomer conventionally used in dental cements. Preferably, a glass is used having a basic surface capable of reacting with acids in a cement reaction. Preferably, the reactive glass is a calcium, strontium or barium fluoroalumosilicate glass. The fluoroaluminosilicate glass powder preferably has a mean particle size of 0.02 to 20 µm and is capable of reacting with polyacidic polymer chains of the hyperbranched polymeric compounds. The particulate reactive inorganic filler is preferably contained in an amount of from 40 to 85 percent by weight, preferably from 50 to 70 percent by weight based on the composition.

The hyperbranched polymeric compounds of the present invention are contained in the dental cement composition preferably in an amount of from 3 percent by weight to 80 percent by weight, preferably in an amount of from 10 percent by weight to 40 percent by weight.

The present invention provides a dental cement composition optionally comprising an organic or inorganic acid selected from the group of tartaric acid, maleic acid, fumaric acid, oxalic acid, phosphoric acid. The acid is used as a retarding agent for adjusting the rate of the glass ionomer reaction.

The dental composition of the invention may further contain a water-soluble or water-swellable polymer or copolymer. Preferably, the water-soluble or water-swellable polymer is selected form the group of polyacrylic acid, polyvinylalcohol, or polyvinylpyrolidone. Preferably, the water-soluble copolymer is obtained by polymerization of at least two different polymerizing monomers in that manner that at least one of the polymerizing monomers contains acidic moieties selected of the group of carboxylic acids, phosphoric acid, phosphonic acid, sulfuric acid, sulfonic acid. In a preferred embodiment, the water-soluble copolymer is obtainable by polymerization of at least two different polymerizing monomers selected of the groups a) monomers such ethylene, propylene, styrene, methylmethacrylate, methylacrylate, butylmethacrylate, vinylalkylether and b) acidic monomers such as acrylic acid, methacrylic acid, vinylphosphonic acid, maleic acid, fumaric acid, maleic acid anhydride, itaconic acid or an anhydride thereof. In a further preferred embodiment of the dental composition, the water-soluble copolymer is a latex.

The dental composition of the invention may further contain additional inorganic fillers widely used for dental composite resins in combination with the reactive inorganic filler. The additional filler preferably has a mean particle size of 0.02 to 10 µm and is incapable of reacting with polyacidic polymer chains of the hyperbranched polymeric compounds by a cement reaction. Examples of the additional filler are colloidal silica, quartz, feldspar, alumina, titania, borosilicate glass, kaolin, talc, calcium carbonate, calcium phosphate, and barium sulfate. Composite fillers obtained by pulverizing inorganic filler-containing polymers may be used as well. These fillers may also be used in admixture.

For increasing the amount of the fluoride ions to be released from a dental composition according to the present invention, the dental cement composition may contain any known water-soluble fluoride compound provided that it does not have any negative effect on the mechanical properties of the cured product of the cement composition. A water-soluble fluoride compound may be a water-soluble metal fluoride such as lithium fluoride, sodium fluoride, potassium fluoride, magnesium fluoride, calcium fluoride, strontium fluoride, barium fluoride, zinc fluoride, aluminum fluoride, sodium monofluorophosphate, fluorostannates, fluorosilicates.

The dental compositions may further contain pigments. In case the dental composition is curable by a combination of a glass ionomer reaction and a polymerization reaction, the dental composition may contain an initiator system, preferably a water-soluble initiator system. The initiator system may be a redox initiator system or a photoinitiator system.

The composition of a typical dental cement composition according to the invention is as follows:

| **Component in the dental cement** | **Percent by weight based on the total composition (preferred range)** |
|---|---|
| Particulate reactive inorganic filler | 40-85 (50-70) |
| Hyperbranched polymeric compounds | 3-80 (5-20) |
| Water | 1-65 (5-45) |
| Additional polyacid | 0- 70 (0-50 and up to 90wt% of the hyperbranched polymeric compounds used) |
| Additional filler | 0-20 (0-10) |

In case the hyperbranched polymeric compounds of the invention contain polymerizable groups, the cement composition of the invention may further contain an initiator system for thermal polymerization or photopolymerisation. Moreover, further polymerisable monomers may be incorporated into the dental cement composition of the invention in an amount of up to 20 percent by weight.

According to the present invention, the hyperbranched polymeric compounds are used for the preparation of dental compositions curable by a cement reaction. The dental composition may be curable by a cement reaction and additionally by a further reaction. Further reactions are polymerization reactions and polyaddition reactions.

The dental composition is a multi-pack, preferably a two-pack composition. The composition may be a paste/paste system, a powder/liquid system, or a liquid/paste system. The composition is designed so as to avoid premature curing of the components. For this purpose, the reactive inorganic filler component and any acid group containing component must be formulated so as to avoid a premature cement reaction. In a first embodiment, the reactive inorganic filler is contained in a first pack and any acid group containing component is contained in a second pack. The first pack may be a powder or a paste. The second pack may be a liquid or paste. In a second embodiment, the first pack is a powder comprising the reactive inorganic filler and a solid polyacid such as polyacrylic acid, and the second pack is a paste or liquid and contains a further acid group containing component.

In a first packaging embodiment which is a powder/liquid kit, a liquid composition containing the hyperbranched polyacid and water is packaged separately from a powdery composition containing the ion-leachable reactive inorganic filler.

In a second packaging embodiment which is a two-paste kit, a first paste composition containing the hyperbranched polyacid, water and a non-reactive filler is packaged separately from a paste composition containing the ion-leachable reactive inorganic filler.

The dental cement composition of the invention may be used in restoring decayed or injured teeth, whereby the cavity of the tooth to be restored is cleaned in a conventional manner, and the cement composition is filled into the cavity of the tooth.

The dental cement composition of the invention may be used in bonding prostheses, such as crowns or inlays to the cavity of a decayed or injured tooth or to an abutment the cavity of the tooth and the surface of the prostheses are cleaned, whereby the cement composition is, applied to the tooth cavity, the abutment surface and/or the prostheses surface, and the prostheses is bonded to the tooth cavity or to the abutment surface.

The invention will now be further illustrated based on the following Examples:

### Example 1

### Synthesis of amino-terminated polyacrylic acid (aet-paa)

A polyacrylic acid with terminal amino groups (aet-paa) was synthesized by aqueous chain transfer polymerization using cysteamine as a functional chain transfer agent and ammonium persulfate as initiator.

A solution of acrylic acid (10.0 g, 0.14 mol) in distilled water (170 mL) was prepared . Then, the monomer solution was deaerated for 1 h with dry nitrogen bubbling before introduction of ammonium persulfate (3.2 g, 0.014 mol) and cysteamine (2.16 g, 0.028 mol) already dissolved separately in 15 ml of water. The temperature was adjusted at 35°C (oil bath) and the reaction was allowed to proceed for 24 h. The final solution was dialyzed (MWCO 1000), then lyophilized and polyacrylic acid containing terminal amino groups was obtained as a white powder. Yield: 8.7 g.

The obtained amino terminated polyacrylic acid (aet-paa) has molecular weight ranging from about 1.000 Daltons to about 3.000 Daltons according to MALDI-TOF MS.

### Example 2 (EB3)

Aet-paa (5.3 g) was placed in a pressure-resistant test tube provided with a magnetic stirring bar. The tube was sealed with a septum, placed in the CEM microwave apparatus by using a program with power of 20W and T=120°C (IR pyrometer) for 10 min. The reaction was performed under temperature control conditions. A yellowish powder was obtained after lyophilization. Yield: 4.4 g.

### Example 3 (EB4)

### Polyacrylic acid (MW 136.900 Da)

Aet-paa (5 g) and polyacrylic acid (5 g) were thoroughly mixed as fine powders in a porcelain cup. Subsequently, the mixture was placed in a pressure-resistant test tube provided with a magnetic stirring bar. The tube was sealed with a septum, placed in the CEM microwave apparatus by using a program with power of 10W and T=105°C (IR pyrometer) for 10 min. The reaction was performed under temperature control conditions. The obtained product was dissolved in distilled water. The final solution was dialyzed (MWCO 8000), then lyophilized and yellowish powder was obtained. Yield: 6.9 g.
Element analysis: C: 47.2%; H: 6.0%; N: 0.6%

### Example 4 (EB5)

Aet-paa (3 g) and polyacrylic acid (6 g) are thoroughly mixed as fine powder in a porcelain cup. Thereupon the mixture was placed in a pressure-resistant test tube provided with a magnetic stirring bar. The tube was sealed with a septum, placed in the CEM microwave apparatus by using a program with power of 10W and T=105°C (IR pyrometer) for 10 min. The reaction was performed under temperature control conditions. The obtained product was dissolved in distilled water. The final solution was dialyzed (MWCO 8000), then lyophilized and yellowish powder was obtained. Yield: 7.1 g.
Element analysis: C: 48.4%; H: 6.3%; N: 0.4%

### Example 5 (EB6)

Aet-paa (6 g) and polyacrylic acid (3 g) are thoroughly mixed as fine powder in a porcelain cup. Thereupon the mixture was placed in a pressure-resistant test tube provided with a magnetic stirring bar. The tube was sealed with a septum, placed in the CEM microwave apparatus by using a program with power of 10W and T=105°C (IR pyrometer) for 10 min. The reaction was performed under temperature control conditions. The obtained product was dissolved in distilled water. The final solution was dialyzed (MWCO 8000), then lyophilized and yellowish powder was obtained. Yield: 6.5 g.
Element analysis: C: 46.9%; H: 6.0%; N: 0.7%

### Application Examples:

The polyacids according to the synthesis examples were incorporated as acid components into dental glass ionomer cements. Accordingly, each hyperbranched polyacid was spatulated with a standard ionomer powder based on zinc strontium calcium phosphor alumino fluorosilicate glass with a powder/liquid ratio of 3.6/1 part by weight.

An unbranched polyacid and a commercially available glass ionomer cement were used as comparative examples.

Bending strength was determined according to ISO 4049 with test specimens having a length of 30 mm. In each case, a mean value for a series of six test specimens was determined. Moreover, compressive strength was determined according to ISO 9917.

The results are shown in the following table

| | Application Example (Invention) |
|---|---|
| Polyacid concentration [wt.%] | 11,0 |
| Bonding Strength [MPa] | 44.5 |
| Compressive Strength [MPa] | 176.2 |

## Claims

1. A process for the preparation of a composition comprising hyperbranched polymeric compounds, which comprises a step of reacting a mixture comprising one or more compounds of the following formula (I):
AR(B)ₙ
wherein
A and B are functional groups,
R is an (n+1)-valent organic group containing one or more thioether groups and
n is an integer of at least 2
**characterized in that**
A is an amino group or a hydroxyl group, and
B is a carboxylic acid group or an ester or anhydride thereof,
under reaction conditions wherein A reacts with B and forms a linking amide group, while A does not react with A and B does not react with B.

2. The process according to claim 1, wherein the one or more compounds of formula (I) comprise a compound obtainable by telomerizing a mixture containing one or more polymerizable unsaturated carboxylic acid monomers or esters or anhydrides thereof with a compound containing a group A and one or more SH-groups.

3. The process according to claim 2, wherein the compound containing a group A and one or more SH-groups is a compound of the following formula (II)
Y-L1-X(L²SH)x
wherein
Y is an amino group or a hydroxyl group;
L¹ is a linking group,
L² is a linking group,
X is a single bond, O S, NR^{a}, -N<, -CR^{a}R^{b}, or -CR^{a}< or >C<,
x is an integer of from 1 to 3 so that the valencies of X is (x+1), and
R^{a} and R^{b} are independent from each other hydrogen, a carboxylic acid group when linked to a carbon atom, or an alkyl group.

4. The process according to claim 2 or 3, wherein the one or more polymerizable unsaturated carboxylic acid monomers are selected from acrylic acid, itaconic acid, maleic acid, fumaric acid or methacrylic acid.

5. The process according to any one of the preceding claims wherein the mixture further comprises a polyacrylic acid molecule or an anydride thereof having an average molecular weight of from 0.5 to 500 kDa.

6. The process according to any one of the preceding claims, which further comprises a step of crosslinking the hyperbranched compounds.

7. A dental composition comprising a hyperbranched polymeric compound obtainable according to the process of any one of claims 1 to 6.

8. The dental composition according to claim 7, which is a dental cement.

9. Use of a composition comprising hyperbranched polymeric compounds, which is obtainable according to any one of claims 1 to 6, for the preparation of a dental cement.
